# EUROPÄISCHE PATENTSCHRIFT

(11) **EP 0 383 130 B1**
(45) Veröffentlichungstag und Bekanntmachung des Hinweises auf die Patenterteilung: **05.10.1994**
(21) Anmeldenummer: 90102187.3
(22) Anmeldetag: 03.02.1990
(51) Int. Cl.: C07C 45/00, C07C 49/83

(54) **Verfahren zur Herstellung von 2,4-Dihydroxybenzophenon**
Process for the preparation of 2,4-dihydroxybenzophenone
Procédé pour la préparation de 2,4-dihydroxybenzophénone

(30) Priorität: 14.02.1989 DE 3904371
(43) Veröffentlichungstag der Anmeldung: 22.08.1990
(73) Patentinhaber: BASF Aktiengesellschaft, 67063 Ludwigshafen (DE)
(72) Erfinder: Neumann, Peter, Dr., D-6800 Mannheim 31 (DE); Aumueller, Alexander, Dr., D-6705 Deidesheim (DE)

(56) Entgegenhaltungen:
- EP-A- 0 032 275
- EP-A- 0 074 272
- US-A- 3 769 349
- CHEMICAL ABSTRACTS, Band 79, 1973, Seite 299, Spalte 1, Zusammenfassung Nr.146215b, Columbus, Ohio, US; & JP-A-73 28 431

## Beschreibung

2,4-Dihydroxybenzophenon ist ein technisch wichtiger UV-Absorber, der zum Schutz von organischem Material vor der Schädigung durch Sonnenlicht und im Kosmetiksektor als Sonnenschutzmittel verwendet wird.

Es ist seit langem bekannt, daß 2,4-Dihydroxybenzophenon durch Umsetzen von Benzotrichlorid mit Resorcin in Wasser hergestellt werden kann (Ber. Dtsch. Chem. Ges. 27, 1998 (1894)). Nachteil dieses Verfahrens ist die geringe Ausbeute und die Bildung von dunkelgefärbten Nebeprodukten, die einen unwirtschaftlich hohen Reinigungsaufwand erfordern. Die DE-OS 22 08 970 beansprucht ein Verfahren zur Herstellung von 2,4-Dihydroxybenzophenon in einem organischen Lösungsmittelgemisch. Nachteilig ist hier die aufwendige Rückgewinnung der Lösungsmittelkomponenten.

Ebenfalls technisch aufwendig ist das in der DE-OS 24 51 037 beschriebene Verfahren, bei dem Fluorwasserstoff als Lösungsmittel verwendet wird.

Weiterhin ist aus dem Stand der Technik die Verwendung von Gemischen aus Wasser und organischen Lösungsmitteln wie Methanol, Essigsäure, Dioxan und/oder Isopropanol bekannt (DD-PS 93 546, DE-OS 20 10 535, US-PS 37 69 349). Neben niedrigen Ausbeuten haben diese Verfahren den Nachteil, daß das Lösungsmittel nur umständlich zurückgewonnen werden kann oder das Abwasser stark durch organische Lösungsmittelrückstände belastet ist.

Aus der Literaturstelle Chemical Abstracts, Bd. 79, 1973, 146 215 b, der die japanische Offenlegungsschrift 73/28431 zugrunde liegt, ist bekannt, 2,4-Dihydroxybenzophenon durch wäßrige Umsetzung von Resorcin mit Benzotrichlorid in Gegenwart von Polyoxyethylenglykolalkylethern oder Polyoxyethylenalkylaminen als Emulgatoren herzustellen. Entsprechend den Beispielen 1 bis 4 der JP-OS liegt die Ausbeute dieser Umsetzung bei 81 bis 88,3 %.

Die EP-A 032 275 offenbart ein Verfahren zur Herstellung von substituierten 2-Hydroxybenzophenonen, beispielsweise von 2,4-Dihydroxybenzophenon aus Resorcin und Benzotrichlorid, in Gegenwart von mindestens 10 Gew.-% n-Octanol als Lösungsmittel.

Aufgabe der vorliegenden Erfindung war es, ein umweltfreundliches und technisch gut durchführbares Verfahren zur Herstellung von 2,4-Dihydroxybenzophenon bereitzustellen.

Die Erfindung betrifft ein Verfahren zur Herstellung von 2,4-Dihydroxybenzophenon durch Umsetzen von Resorcin und Benzotrichlorid in Wasser, das dadurch gekennzeichnet ist, daß das Reaktionsgemisch - bezogen auf Wasser - 0,05 bis 5 Gew.%, vorzugsweise 0,05 bis 2 Gew.-%, eines gesättigten oder ungesättigten Fettalkohols (Alkanols), eines EO-Adduktes an Alkylphenol, einer Fettsäure in Form der Salze, eines Polyethylenglykols, eines Schwefelsäurehalbesters eines Alkylphenol-EO-Adduktes, eines Fettsäureesters, einer Alkylbenzolsulfonsäure oder eines Gemisches aus diesen Mitteln als Hilfsmittel in der Funktion als grenzflächenaktive Verbindung, Dispergiermittel oder Emulgator enthält.

Überraschend war, daß bereits ein geringer Zusatz der genannten Hilfsmittel bei der Reaktion von Benzotrichlorid mit Resorcin in Wasser ein reines Produkt in hoher Ausbeute liefert.

Als Hilfsmittel kommen die genannten grenzflächenaktiven Verbindungen (Tenside), Dispergiermittel oder Emulgatoren in Betracht. Diese können nichtionisch oder ionisch und nieder- oder hochmolekular sein. Beispielsweise sind zu nennen:
C₈-C₂₀-Alkenole, C₈-C₂₀-Alkanole, Polyvinylalkohole, Fettsäuren, Fettsäureester, Ethylenoxidaddukte an C₁-C₂₀-Alkylphenole, C₁-C₂₀-Alkanole, an C₂-C₈-Alkan- oder Alkendiole, C₃-C₈-Alkantriole, an C₁₀-C₂₅-Alkan- oder Alkensäuren, an Amide von C₁₀-C₂₅-Alkan- oder Alkensäuren, an aliphatische oder cycloaliphatische Amine, saure Phosphorsäureester oder Schwefelsäurehalbester der obengenannten Alkenole, Alkanole, und EO-Addukte, Ester der phoshorigen Säure, Phosphonate, Phosphinate, Alkylnapththalinsulfonsäuren, Sulfobernsteinsäureester, C₅-C₂₀-Alkylbenzolsulfonsäuren, Polystyrolsulfonsäure, C₁₀-C₂₀-Alkan- und Alkensulfonsäuren, Mono-C₈-C₂₀-Alkyl-tri-C₁-C₄-alkylammoniumsalze, Di-C₈-C₂₀-alkyl-di-C₁-C₄-alkylammoniumsalze und C₁₀-C₂₀-Alkylpyridiniumsalze.

Im einzelnen sind z.B. zu nennen:
Oleylalkohol, Polyethylenglykol (M̅ 200, 400, 1000) Schwefelsäurehalbester des EO-Adduktes an C₉-Alkylphenol, Schwefelsäurehalbester des EO-Adduktes an C₁₆-C₁₉-Oxoalkohole und C₁₂-C₁₄-Alkanole, Dodecylbenzolsulfonsäure, C₁₄-Alkylsulfonsäure, C₁₇-Alkylsulfonsäure, C₁₂-, C₁₄- und C₁₈-Fettalkoholsulfat, EO-Addukte an Alkanole mit 12, 14, 15, 16 und 18 C-Atomen, EO-Addukte an Hexyl-, Nonyl- und Dodecylphenol, Stearinsäurepolyglykolester, Ölsäurepolyglykolester, Diölsäurepolyglykolester, Ölsäureamidpolyglykolether, Cocosfettsäuremonoethanolamid, Cocosfettsäurediethanolamid, Stearyl-, Palmityltrimethylammoniumsalze, Distearyl-dimethylammoniumsalze, Dodecyl-benzyl-dimethylammoniumsalze, Stearyl und Dodecylpyridiniumsalze.

Die sauren Phosphorsäureester und die Schwefelsäurehalbester (Sulfate) werden in der Regel in Form der löslichen Salze wie der Alkalimetall oder der Aminsalze angewendet, die Ammoniumsalze in Form der Bromide, Sulfate, Acetate, insbesondere der Chloride.

Das Verfahren gemäß der Erfindung soll durch die folgenden Beispiele zusätzlich erläutert werden.

### Beispiel 1

In einem 2-l-Gefäß werden 1000 ml Wasser vorgelegt. Man trägt unter Rühren 220 g (= 2,0 mol) Resorcin ein, das sich auflöst. Anschließend werden 2,12 ml (= 2,5 g) cis-9-Octadecen-1-ol (= Oleylalkohol) zugegeben. Innerhalb von 1,5 Stunden werden 294 ml (= 2,0 mol) Benzotrichlorid zugetropft. Dabei tritt eine stark exotherme Reaktion auf. Dabei muß so langsam zugetropft werden, daß die Reaktionstemperatur zwischen 40 und 50°C gehalten wird.

Nach dem Zutropfen wird 1 h auf 70°C erhitzt, man schaltet die Heizung aus, saugt bei 60°C ab und wäscht mit heißem Wasser nach.

Nach Trocknen im Vakuum bei 80°C erhält man 420 g (98 % d. Th.) rohes 2,4-Dihydroxybenzophenon als hellgelboranges Pulver vom Schmp. 139 - 140°C.

Verwendet man anstelle von 1000 ml Wasser 750 ml, so erhält man das gleiche Ergebnis.

### Beispiele 2 bis 12

Es wurde wie in Beispiel 1 verfahren, jedoch wurden die in Spalte 2 der folgenden Tabelle angegebenen Hilfsmittel in der in Spalte 3 angegebenen Menge angewendet. Die Ausbeute und der Schmelzpunkt der Verfahrensprodukte sind in den Spalten 4 und 5 der folgenden Tabelle angegeben.

Bei den Beispielen 9 bis 12 wurde ein 4 l-Gefäß verwendet.

**Tabelle 1**

| Herstellung von 2,4-Dihydroxybenzophenon | | | | |
|---|---|---|---|---|
| Bsp. | Hilfsmittel | Menge Hilfsmittel [g] | Ausbeute [% d. Th.] | Schmelzpunkt [°C] |
| 2 | Oleylalkohol | 5,00 | 96 | 139 - 141 |
| 3 | 1-Heptanol | 1,73 | 96 | 136 - 138 |
| 4 | 1-Dodecanol | 5,00 | 97 | 136 - 138 |
| 5 | Ethylenglykol | 2,24 | 94 | 136 - 138 |
| 6 | 1,3-Propandiol | 2,50 | 93 | 138 - 140 |
| 7 | A | 5,00 | 97 | 134 - 136 |
| 8 | A | 10,00 | 96 | 138 - 140 |
| 9 | B ²) | 3,00 | 95 | 139 - 141 |
| 10 | B ²) | 6,25 | 93 | 140 - 141 |
| 11 | C ²) | 12,50 | 95 | 135 - 137 |
| 12 | D ²) | 12,50 | 94 | 137 - 139 |
| A = Polyethylenglykol (mittleres Molekulargewicht 200) B = Schwefelsäurehalbester vom EO-Addukt an C₉-Alkylphenol C = Alkanolaminsalz der Dodecylbenzolsulfonsäure D = Schwefelsäurehalbester vom EO-Addukt an C₁₆-C₁₉-Oxoalkoholgemisch | | | | |

| | | | | |
|---|---|---|---|---|
| ²) Verwendung von 2 l Wasser als Solvens | | | | |

## Patentansprüche

1. Verfahren zur Herstellung von 2,4-Dihydroxybenzophenon durch Umsetzen von Resorcin und Benzotrichlorid in Wasser, dadurch gekennzeichnet, daß das Reaktionsgemisch - bezogen auf Wasser - 0,05 bis 5 Gew.-% eines gesättigten oder ungesättigten Fettalkohols, eines Ethylenoxi-Adduktes an Alkylphenol, einer Fettsäure in Form der Salze, eines Polyethylenglykols, eines Schwefelsäurehalbesters eines Alkylphenol-Ethylenoxi-Adduktes, eines Fettsäureesters, einer Alkylbenzolsulfonsäure oder eines Gemisches aus diesen Mitteln als Hilfsmittel in der Funktion als grenzflächenaktive Verbindung, Dispergiermittel oder Emulgator enthält.

2. Verfahren gemäß Anspruch 1, dadurch gekennzeichnet, daß die Menge an Hilfsmittel 0,05 bis 2 Gew.-%, bezogen auf Wasser, beträgt.

## Claims

1. A process for the preparation of 2,4-dihydroxybenzophenone by reacting resorcinol and benzotrichloride in water, wherein the reaction mixture contains from 0.05 to 5% by weight, based on water, of a saturated or unsaturated fatty alcohol, of an ethylene oxide adduct with alkylphenol, of a fatty acid in the form of the salts, of a polyethylene glycol, of a sulfuric half-ester of an alkylphenol/ethylene oxide adduct, of a fatty ester, of an alkylbenzenesulfonic acid or of a mixture of these agents as an assistant acting as a surfactant compound, dispersant or emulsifier.

2. A process as claimed in claim 1, wherein the amount of assistant is from 0.05 to 2% by weight, based on water.

## Revendications

1. Procédé de préparation de la 2,4-dihydroxybenzophénone par la réaction de la résorcine et du benzotrichlorure dans l'eau, caractérisé en ce que le mélange réactionnel - par rapport à l'eau - contient 0,05 à 5% en poids d'un alcool gras saturé ou insaturé, d'un adduit de l'oxyde d'éthylène et d'un alkylphénol, d'un acide gras sous forme des sels, d'un polyéthylèneglycol, d'un hémiester de l'acide sulfurique, d'un adduit d'alkylphénol et d'oxyde d'éthylène, d'un ester d'acide gras, d'un acide alkylbenzènesulfonique, ou d'un mélange de ces agents, à titre d'adjuvant dans la fonction de composé tensioactif, de dispersif ou d'émulsif.

2. Procédé suivant la revendication 1, caractérisé en ce que la proportion d'adjuvant varie de 0,5 à 2% en poids par rapport à l'eau.
